# FASCICULE DE BREVET EUROPEEN

(11) **EP 1 794 320 B1**
(45) Date de publication et mention de la délivrance du brevet: **04.06.2008**
(21) Numéro de dépôt: 05805383.6
(22) Date de dépôt: 23.09.2005
(51) Int. Cl.: C12Q 1/68

(54) **MÉTHODE POUR GÉNÉRER DES TRANSCRITS**
VERFAHREN ZUR ERZEUGUNG VON TRANSKRIPTEN
METHOD FOR GENERATING TRANSCRIPTS

(30) Priorité: 01.10.2004 FR 0452228
(43) Date de publication de la demande: 13.06.2007
(73) Titulaire: BIOMERIEUX, 69280 Marcy-L'Etoile (FR)
(72) Inventeur: MALLET, François, F-69100 VILLEURBANNE (FR); ORIOL, Guy, F-42400 SAINT-CHAMOND (FR); PICHON, Jean-Philippe, F-69007 LYON (FR)
(74) Mandataire: Denjean, Frédérique
(86) Numéro de dépôt international: PCT/FR2005/050775
(87) Numéro de publication internationale: WO 2006/054003

(56) Documents cités:
- WO-A-99/25868
- WO-A-99/43850
- WO-A-02/072773
- US-B1- 6 589 734

### SEQUENCE LISTING

<110> bioMérieux SA
<120> Méthode pour générer des transcrits
<130> unknown<160> 29
<170> PatentIn version 3.1
<210> 1
<211> 737
<212> RNA
<213> HML.4
<400> 1
   gucacuguca agccuccaga ugccaucccu uugacuugaa aacucagcca guuugggugg 60
   aucaguggcc gcucccaaaa aauaagcugg aggcgcucca uaauuuaguc cuggaacagu 120
   uagaauuggg acacauugag gaaucuuucu cuccauggaa uucacuuguc uuuguuaucc 180
   aaaagaaauc uggggaaaac agagaaugcu cacucaucuu agggcaguua augcuguacu 240
   ucaaccucug gggacauuac aaucuggcuu acccucccgc ucuaugcucg cugaguauug 300
   gccucuaauc cucauagauc uuaaagauug cuuuuuuaac auuccacugg ccucucagga 360
   cuuugaaaag uuugcuuuua uggucccuuc ccucaacaau gucgcucagg cuacaugcua 420
   cuauuggaaa guccuaccac aaggcaugcu uaauaguccc acuauuuguc aguauuuugu 480
   ggggcgugug cuucaaccug ucagggauca guuuccccga uguuacaucg uucacuacau 540
   ggaugaucuc cucugcacag cccccccaua caccauuuug auuuccugcu uuucugugau 600
   ucaacaggcc auuucagaag cagguuugac uauugcacca gaaaaaauuc aaacuaccuc 660
   ucauuuucaa uauuugggca ugcaguugga agacaagcug auuacaccac aaaaaguuca 720
   gcuuaggaga gacgccu 737
<210> 2
<211> 857
<212> RNA
<213> HERV.E4.1
<400> 2
   ggccaagaga uaagaccagc ucuggcuaag cgguggccaa gagugugggc ggaagacaac 60
   ccuccagggu uggcagucaa ccaagccccc gugcuuauag aaguuaagcc ugggguccag 120
   ccgguuaggc aaaaacagua cccgguccuc agagaagcuc uugaagguau ccagguccau 180
   cucaagugcc uaagaaccuu uagaauuaua guuccuuguc agucuccaug gaacacuccc 240
   cuccugccug uucccaagcc ugggaccaag gacuacaggc cgguacagga uuugcgcuug 300
   guuaaucagg cuacagugac uuuacaucca acaguaccua accuguacac auugcugggg 360
   uugcugccag cugaggacag cugguucacc ugcuuggacc ugaaagaugc cuucuuuagc 420
   aucagauuag ccccugagag acagaagcug uuugccuuuc agugggaaga uccagaguca 480
   ggugucacua cucaauacac uuggacccag ccuccccaaa gguucaagaa cucccccacc 540
   aucuuugggg aggcguuggc ucgagaccuc cagaaguuuc ccaccagaga ccuaggcugc 600
   guguugcucc aguacguuga ugaccuuuug cugggacacc ccacggcagu cgggugcgcc 660
   aagggaacag augcucuacu ccggcaccug gaggacugug gguauaaggu guccaagaaa 720
   aaaagcucag aucugccgac agcagguaug uuacuuggga uuuacuaucc aacaggggga 780
   gcacagccug ggaucagaaa gaaagcaggu cauuuguaau cuaccggagc cuaagaccag 840
   aaggcaggug agagaau 857
<210> 3
<211> 62
<212> DNA
<213> Articial
<400> 3
   taatacgact cactataggg agatctagca gcataggggg ggctgtgcag aggagatcat 60
   cc 62
<210> 4
<211> 62
<212> DNA
<213> HERV-E4
<400> 4
   taatacgact cactataggg agatctagca gcatacagca aaaggtcatc aacgtactgg 60
   ag 62
<210> 5
<211> 22
<212> DNA
<213> HML.4
<400> 5
   caacctgtca gggatcagtt tc 22
<210> 6
<211> 22
<212> DNA
<213> HERV.E4.1
<400> 6
   ctcgagacct ccagaagttt cc 22
<210> 7
<211> 828
<212> RNA
<213> HML-2
<400> 7
   cacuguagag ccuccuaaac ccauaccauu aacuuggaaa acagaaaaac uggugugggu     60
   aaaucagugg ccacuaccaa aacaaaaacu ggaggcuuua cauuuauuag caaaugaaca    120
   guuagaaaag ggucauauug agccuucauu cucgccuugg aauucuccug uguuuguaau    180
   ucagaagaaa ucaggcaaau ggcauauguu aacugacuua agggucguaa acgccguaau    240
   ucaacccaug gggccucucc aacccggguu gcccucuccg gccaugaucc caaaagauug    300
   gccuuuaguu auaauugauc uaaaggauug cuuuuuuacc aucccucugg cggagcagga    360
   uugcgaaaaa uuugccuuua cuauaccagc cauaaauaau aaagaaccag ccaccagguu    420
   ucaguggaaa uuguuaccuc agggaaugcu uaauagucca acuauuuguc agacuuuugu    480
   aggucgagcu cuucaaccag uuagagacaa guuuucagac uguuauauua uucauuauau    540
   ugaugauauu uuaugugcua cagaaacgag agauaaauua auugacuguu auacauuucu    600
   gcaagcagag guugccaaug caggacuggc aauagcaucu gauaagaucc aaaccucuac    660
   uccuuuucau uauuuaggga ugcagauaga aaauagaaaa auuaagccac aaaaaauaga    720
   aauaagaaaa gacacauuaa aaacacuaaa ugauuuucaa aaauugcugg gagauauuaa    780
   uuggauucgg ccaacucuag gcauuccuac uuaugccaug ucaaauuu                 828
<210> 8
<211> 870
<212> RNA
<213> HML-5
<400> 8
   uauccaauuu ggguagacca guggccuuua aagggagaga aauugcaaag agcccaugag     60
   uuuguugaag agcaauuaaa agccagccau auagaaccau caaaaagacc uuggaauuca    120
   cccauuuuca ucauucccaa aaagucuggu aaauagagac uuuugcauga cuaacaugcu    180
   aucaaugcua auuugaaacc uaugggaccc cuugagcagg uguucuccuc cccuucagug    240
   auuccuugag auuggcauau aauaguuauu ggcuuaaaau acugcauuug uacuauuccc    300
   uuugcagaac aggacagaga aaaauuugug uuuauaauac cagcuauaaa uaacgaaagg    360
   ccagcucccc gauuucacug gaaagugcuu ccugaaggga ugcuaaacag uccuaccaug    420
   ugucaguauc auguaaauca agcuuugcuc cucaguagaa aagaauuucc uaauugcaag    480
   acuauucauu uuauggauga uauuuuacua gcagccccaa uggauccagc acuuuuaagu 540
   uuaugugccu cuguuguaaa gaauacacag uuaagagguu uaaucauagc accugaaaaa 600
   guacagaugu ccucuccuug gaaauaucuu ggguacauac uaacuucgug gucaguaaga 660
   ccucaaaagg uuaaauuaaa uacuagcaac uuacacaccu uaaaugauua ucagaaauua 720
   cuaggcaaua uuaacuggcu uugccccacc uugggcauaa cuacugauaa guuacagaac 780
   cuguuuucua ucuuaaaaga caaugcugcu cuagacucuc ccagguauuu aacuccugca 840
   gcacaaaggg aaauugagga aguagagcac 870
<210> 9
<211> 866
<212> RNA
<213> HERV-H
<400> 9
   cacccuuacc cugcucaaug ccaauauccc aucccacagc augcuuuaaa aggauuaaag 60
   ccuguuauca cucgccugcu acagcauggg cuucuagaac cuauaaacuc ucuuuucaau 120
   ucccccauuu uaccugucca aaaaccggau aagucuuaca gacuaguuca gaaucugcgu 180
   cuuaucaacc aaauuguuuu gccuauccac ccuguggugc ccaaccugua cacucuuuug 240
   uccucaauac cuuccuccac aacucacuau uccgugcuug aucuuaaaga ugguuuuuuc 300
   acuauucucc ugcacuccuc gucccagccu cucuuugcuu ucaccuggac ugacccugac 360
   acccaucagu cccagcagcu uaccuggacu gugcugccgc aagguuucag ggacagcccu 420
   cguuacuuca gccaagcucu uucucaugau cuacuuuccu uccaccccuc cgcuucucac 480
   cuuauucaau auauugauga gcuucuucuu uguagccccu ccuuugaauc uucucaauaa 540
   gacacacuuc ugcuccuuca gcauuuauuc uccaaaggau aucggguauc ccccuccaaa 600
   gcucaaauuu cuucuccauc cguuaccuac cucggcauaa uucuucauaa gaacacacgu 660
   gcucucccug ccgaccgugu cugacuaauc ucucaagccc caaccccuuc uacaaaacaa 720
   caacuccuuu ccuuccuggg cgugguugga uacuuucgcc uuuggauacc ugguuuugcc 780
   auccuaacaa aaccauuaua uaaacucaca aaaggaaacu uagcugaccc cauagauccu 840
   aaauccuuuc cccacuccuc uuucug 866
<210> 10
<211> 773
<212> RNA
<213> ERV-9
<400> 10
   cugaagcucu uaaaggauua caggauauug uuaaacauuu aaaagcucaa ggcuuaguaa 60
   ggaaaugcag cagucccugc aacaccccaa uucuaggagu acaaaaacca aauggucacu 120
   ggagacuagu gcaagaucuu agacucauca augaggcagu aauuccucua uauccaguug 180
   uacccaaccc cuauacccug cuuucucaaa uaccagagga agcagaaugg uucaugguuc 240
   uggaccucaa ggaugccuuc uucuguuucc ccugcacucu gacucccagu uucuguuugc 300
   cuuugaggau cccacagacc acacguccca acuuacaugg auggucuugc cacaaggguu 360
   uagggauagc ccucaccugu uuggucaggc acuggcccaa gaucuaggcc acuucucaag 420
   uccaggcacu uugguccuuc aguaugugga ugauuuacuu uuggcuacca guucagaagc 480
   cucaugccag caggcuacuc uagaucucuu gaacuuucua ccuaaucaag gguacaaggc 540
   aucuagguca aaggugcagc uuugcuuaca gcaggcuaaa uaucuaggcc uaaucuuagc 600
   cagagggacc agggcccuca gcaaggaaug aauacagccu auacuggcuu auccuugccc 660
   uaagacauua aaacaguugc agggguuccu uggaaucacc ggcuuuugcc gacuauggau 720
   cccuggauac agugagauag ucaggccccu ccauacucua aucaaggaga ccc 773
<210> 11
<211> 815
<212> RNA
<213> HERV-W
<400> 11
   gacccaaggc ccaacaagga cuccaaaaga uuguuaagga ccuaaaagcc caaggccuag 60
   uaaaaccaug caguaacccc ugcaguacuc caauuuuagg aguacagaaa cccaacagac 120
   aguggagguu agugcaagau cucaggauua ucaaugaggc uguuguuccu cuauagccag 180
   cuguaccuag cccuuauacu cugcuuuccc aaauaccaga ggaagcagag ugguuuacag 240
   uccuggaccu ucaggaugcc uucuucugca ucccuguaca uccugacucu caauucuugu 300
   uugccuuuga agauacuuca aacccaacau cucaacucac cuggacuuuu uaccccaagg 360
   uucagggaua guccccaucu auuugccagg cauuagccca agacuugagc caauccucau 420
   accuggacac uugucuucgg uagguggaug auuuacuuuu ggccgcccau ucagaaaccu 480
   ugugccauca agccacccaa gcgcucuuca auuuccucgc uaccuguggc uacaugguuu 540
   ccaaaccaaa ggcucaacuc ugcucacagc agguuacuua gggcuaaaau uauccaaagg 600
   caccagggcc cucagugagg aacacaucca gccuauacug gcuuauccuc aucccaaaac 660
   ccuaaagcaa cuaaggggau uccuuggcgu aauagguuuc ugccgaaaau ggauucccag 720
   guauggcgaa auagccaggu cauuaaauac acuaauuaag gaaacucaga aagccaauac 780
   ccauuuagua agauggacaa cugaaguaga agugg 815
<210> 12
<211> 856
<212> RNA
<213> HERV-R
<400> 12
   aucauaaucc aaugccaguc acccuggaac acuccacuuu ugcugguaca aaaaccauug     60
   ccuggaccag gaucugauga guauauacug gugcagggcu ugcaugcugu aaaccaagcc    120
   acggugacca uacauccagu aguaccaaac cuguauacuu uaaugggacu uauucuagca    180
   agugccaccu gguuuacagu ccuggacuua aaggaugcuu uccucugucu cuaccuggca    240
   ccaguuaguc agcccaucuu ugcauuuuaa ugggacaauu cagucacagg cacaggggga    300
   cagcucgccu ggacuagucu cccacaaggg uucaagaauu cucccacaau cuuuggggaa    360
   gcacuggccu cagaccucaa ggcauacacc ccaccaaaug acaacugcgc cuuguugcag    420
   uacauagaca acccuucuuu uggcagcccc aaccccaaga ggacuguaac ugggggaacc    480
   caggaccucc uccaucucuu augggaaagc aggguauaga guauccaaaa agaaggccca    540
   aauuugccau gaaaagguua aauauuuagg cuucauagua agccaagggg aacgcuggcu    600
   uggccaugga ugaaagcaag ccauuugugc acuuccaacu ccaaccaccu ggcgccaaau    660
   aggggaauuc uuaggggcag caggguucug ccauaucugg aucccaaauu ucucacuuau    720
   agccaggccc uuauaugaag ccacaaggga gggugaaaag gaaccccucc ucugaaaggc    780
   ugaccagaag aagguguuua aacaaaucaa agaagcucua acucaggcuc cagccuuagg    840
   acugccagau acuacu                                                    856
<210> 13
<211> 819
<212> RNA
<213> HERV-L
<400> 13
   uuaauggagu uauggcucag gucugacuua cagcaggucc aguggguccc uggacucauc 60
   cuguguucau uuucccagug ccagaaugca uaauuggcau ugucauacuu agaggcuggc 120
   agaaccccca cauugauucu gugacuggua aggugagggc uauuauggug ggaaaggcca 180
   aauggaagcc auuggagcug ccuuuaccua ggaaaauagu aaaucaaaaa cauuaucacc 240
   acccuggagg gauugcagag auuagugcca ccaucaagga cuugaaaaau gcaggggugg 300
   cgauucccau aacauccuug uucaacucuc cuuuuuggcc ugugcagaag acagauggau 360
   cuuggagaau gagaguggau uaucauaagc uuaaccaagu ggugacucca auugcagcug 420
   cuauacaaga ugugguuuuc auugcucaag caaauuaaua caucuccugg uaccuuguau 480
   gcagccauug acuuggcaaa uggccuuuua cccauuccau aagccccacc agaagcgauu 540
   ugccuucagc uggcaaggcc agcaauguau cuuuacuguc cuacuucagg gguauaucaa 600
   cucuccggcu uugugucaua aucuuauuca gagugaucuu gaucacuuuu cacugccaca 660
   agauaucaca cugguccauu acauugaugg cauuauguug auuggaucca augagcaaga 720
   aguagcaaac acacuggacu uauuggugag acauuugcau gccauaggau gggaaauaaa 780
   uccaaauaaa auucacgcac ccucuaccuc aguaaaauu 819
<210> 14
<211> 50
<212> DNA
<213> HML-2
<400> 14
   aattctaata cgactcacta tagggagaca cataaaatat catcaacata 50
<210> 15
<211> 50
<212> DNA
<213> HML4
<400> 15
   aattctaata cgactcacta tagggagatg cagaggagat catccatgta 50
<210> 16
<211> 50
<212> DNA
<213> HML-5
<400> 16
   aattctaata cgactcacta tagggagact agtaaaatat catccataaa 50
<210> 17
<211> 50
<212> DNA
<213> HERV-H
<400> 17
   aattctaata cgactcacta tagggagaaa agtagaaggt catcaatata 50
<210> 18
<211> 51
<212> DNA
<213> ERV-9
<400> 18
   aattctaata cgactcacta tagggagaag taaatcatcc acatactgaa g 51
<210> 19
<211> 50
<212> DNA
<213> HERV-W
<400> 19
   aattctaata cgactcacta tagggagaaa gtaaatcatc cacgtaccga 50
<210> 20
<211> 50
<212> DNA
<213> HERV E4.1
<400> 20
   aattctaata cgactcacta tagggagacc agcaaaaggt catcaacgta 50
<210> 21
<211> 50
<212> DNA
<213> HERV-R
<400> 21
   aattctaata cgactcacta tagggagacc aaaagaaggt tgtctatgta 50
<210> 22
<211> 50
<212> DNA
<213> HERV-L
<400> 22
   aattctaata cgactcacta tagggagatc arcataatgy catcaatgta 50
<210> 23
<211> 24
<212> DNA
<213> HML-2
<400> 23
   caaccagtta gagacaagtt ttca 24
<210> 24
<211> 23
<212> DNA
<213> HML-5
<400> 24
   ttgctcccca gtagaaaaga att 23
<210> 25
<211> 25
<212> DNA
<213> HERV-H
<400> 25
   gacagccccc attacttcag tcaag 25
<210> 26
<211> 22
<212> DNA
<213> ERV-9
<400> 26
   ccaagatcta ggccacttct ca 22
<210> 27
<211> 21
<212> DNA
<213> HERV-W
<400> 27
   catctatttg gccaggcatt a 21
<210> 28
<211> 20
<212> DNA
<213> HERV-R
<400> 28
   agacctcaag gcatacaccc 20
<210> 29
<211> 23
<212> DNA
<213> HER-L
<400> 29
   gctttgtgtc ataatcttat tcg 23

## Description

La présente invention concerne une méthode pour l'amplification de séquences d'acide nucléique cibles.

Il est souvent nécessaire, dans les technologies relatives aux acides nucléiques et au matériel génétique, de déterminer si un gène, une partie de gène ou une séquence nucléotidique est présent dans un organisme vivant, un extrait cellulaire de cet organisme ou tout autre un échantillon biologique. L'intérêt de la recherche de séquences nucléotidiques spécifiques est immense, notamment pour la détection d'organismes pathogènes, la détermination de la présence d'allèles, la détection de la présence de lésions dans un génome hôte et la détection de la présence d'un ARNm particulier ou de la modification d'un hôte cellulaire. De nombreuses maladies génétiques peuvent ainsi être diagnostiquées par l'analyse, et même la quantification, de l'expression de certains gènes. La quantification de l'expression de ces gènes est également fondamentale.

Différents types de méthodes de détection des acides nucléiques sont décrits dans la littérature. Ces méthodes, et particulièrement celles qui requièrent la détection de polynucléotides, reposent sur les propriétés d'appariement des brins complémentaires d'acides nucléiques dans les duplex ADN-ADN, ADN-ARN et ARN-ARN par l'établissement de liaisons hydrogène entre les bases adénine et thymine (A-T) et les bases guanine et cytosine (G-C) de l'ADN double brin, ou encore entre les bases adénine et uracile (A-U) dans les duplex ADN-ARN ou ARN-ARN. L'appariement de brins d'acides nucléiques est communément appelée "hybridation d'acides nucléiques" ou simplement "hybridation".

D'une manière générale, après avoir identifié la séquence spécifique d'un organisme ou d'une maladie qui doit être analysée, il convient d'extraire les acides nucléiques d'un échantillon, et de déterminer si cette séquence (aussi appelée "séquence d'intérêt") est présente. De nombreuses méthodes de détection ont été développées dans ce but.

La méthode la plus directe pour détecter la présence d'une séquence d'intérêt dans un échantillon d'acides nucléiques est d'obtenir une "sonde" dont la séquence est suffisamment complémentaire d'une partie de l'acide nucléique cible pour s'hybrider à celui-ci. La sonde ainsi synthétisée peut être mise en présence d'un échantillon contenant des acides nucléiques, et si la séquence d'intérêt est présente, la sonde s'hybridera et formera un produit de réaction. En l'absence de séquence d'intérêt et en évitant tout phénomène d'hybridation non spécifique, aucun produit de réaction ne sera formé. Si la sonde synthétisée est couplée à un marqueur détectable, le produit de réaction peut être détecté en mesurant la quantité de marqueur présent. Le transfert de type Southem (Southem E.M, J. Mol. Biol., 98, 503 (1975)) ou Northem ou la technique du Dot blot ou l'hybridation sandwich (DUNN A.R. et HASSEL J.k, Cell, 12, 23, (1977)) constituent des exemples de méthodes utilisables.

La principale difficulté de cette approche est, cependant, qu'elle n'est pas directement applicable aux cas où le nombre de copies de la séquence d'intérêt présente dans un échantillon est faible. Dans ces conditions, il est difficile de distinguer un signal significatif supérieur au bruit de fond de la réaction (c'est à dire de distinguer la fixation spécifique d'une sonde sur sa séquence d'intérêt de la fixation non spécifique entre la sonde et une séquence différente de la séquence d'intérêt). Une des solutions à ce problème consiste à augmenter le signal de détection par une réaction supplémentaire. En conséquence, diverses méthodes ont été décrites afin d'accroître la puissance de détection de ces techniques d'hybridation. Ces méthodes dites "d'amplification" peuvent être classés en trois catégories: l'amplification de cible, de sonde ou de signal. Les articles de Lewis (1992, Genetic Engineering News 12:1-9) d'une part, et d'Abramson et Myers (1993, Curer. Opus . Biotechnol. 4 : 4147), d'autre part, constituent de bonnes revues générales de ces méthodes.

L'amplification de cible consiste à multiplier de manière spécifique un fragment d'acide nucléique présent dans un échantillon. Elle permet d'augmenter considérablement le nombre de copies d'une séquence nucléique cible à détecter.

Les techniques d'amplification de cible décrites dans la littérature reposent principalement soit sur la répétition de cycles de synthèse d'ADN in vitro par élongation d'amorces nucléotidiques hybridées sur la séquence cible à amplifier par une ADN polymérase ("Réaction de polymérisation en chaîne", dite PCR: voir les brevets US 4 683 195, US 4 683 202 et US 4 800 159; EP 201 184; la méthode dite"Repair Chain Reaction", ou RCR: voir demande de brevet WO 90/01069; la méthode dite d'amplification par déplacement de brin, ou "Strand Displacement Amplification" (SDA) : voir brevet EP O 497 272; la méthode d'amplification par déplacement de brin utilisant une exonucléase, ou "exonuclease-mediated strand displacement amplification" : voir brevet EP 500 224), soit sur la répétition de cycles de synthèse d'ARN in vitro, par réaction de transcription à l'aide d'une ARN polymérase.

Plusieurs de ces méthodes d'amplification de cible basées sur l'amplification de transcrits ont été décrites. La méthode dite TAS, décrite dans la demande de brevet WO 88/10315, consiste en la répétition d'un cycle de trois étapes. La première étape permet de synthétiser un ADNc à partir d'ARN en présence d'une transcriptase inverse et d'une amorce désoxynucléotidique contenant une séquence spécifique de promoteur d'ARN polymérase phagique. Suite à la dénaturation thermique de l'hétéroduplex ARN/ADNc, le brin monocaténaire d'ADNc est répliqué par la transcriptase inverse en présence d'une amorce oligonucléotidique anti-sens. L'homoduplex d'ADN ainsi obtenu lors de cette deuxième étape contient un promoteur double brin sur lequel une ARN polymérase ADN-dépendante phagique peut se fixer. La troisième étape est une transcription, conduisant à l'obtention de 30 à 1000 molécules d'ARN par matrice qui pourront à leur tour servir de matrice pour la synthèse d'ADNc et poursuivre ainsi le cycle d'amplification (DAVIS et al., 1990. J. Infect. Dis.162:13-20).

Il existe différentes méthodes dérivées de la TAS, dont la réplication de séquence auto-entretenue, dite "Self-Sustained Sequence Replication" (ou 3SR), décrite dans la demande de brevet WO 90/06995 et le brevet EP O 373 960, la méthode dite "Nucleic Acid Sequence-Based Amplification" (ou NASBA) décrite dans la demande de brevet WO 91/02818 et le brevet Européen EP 329 822 et la méthode de réplication de séquence à l'aide d'une seule amorce dite "Single Primer Séquence Replication" (ou SPSR) décrite dans le brevet US 5 194 370.

Ces méthodes possèdent en commun l'association de trois activités enzymatiques : ADN polymérase ARN- et ADN-dépendante (transcriptase inverse), ribonucléase H (RNase H, enzyme d'Escherichia coli et/ou activité enzymatique associée de la transcriptase inverse) et ARN polymérase ADN-dépendante (ARN polymérase du bactériophage T7). Ces méthodes sont basées sur le même principe et s'effectuent à une température fixe (de 37 à 45°C), selon un processus continu de réactions de transcription inverse et de transcription afin de répliquer une cible d'ARN par l'intermédiaire d'ADNc. Comme dans le cas de la TAS, un site de fixation d'une ARN polymérase (phage T7) est introduit dans l'ADNc par l'amorce utilisée pour l'étape de transcription inverse. Néanmoins, la dénaturation de l'hétéroduplex ARN/ADNc est effectuée de façon isotherme par hydrolyse spécifique de l'ARN de cet hétéroduplex par l'activité RNase H. L'ADNc libre est alors répliqué à partir d'une seconde amorce oligonucléotidique par la transcriptase inverse. L'homoduplex ADN/ADN est transcrit en ARN par l'ARN polymérase T7 et cet ARN peut à nouveau servir de matrice pour le cycle suivant.

Une autre méthode, dite "Ligation Activated Transcription" (ou LAT) décrite dans le brevet US 5 194 370, utilise les mêmes activités enzymatiques que les méthodes 3SR, SPSR et NASBA et fonctionne sur le même cycle. Elle diffère cependant par le mode d'installation d'une séquence promotrice qui dans ce cas est introduite sur l'extrémité de l'ADNc par ligation d'une structure tige-boucle contenant le promoteur, en présence d'une ADN ligase.

Il existe également deux autres méthodes d'amplification de cible basées sur l'amplification de transcrits décrites dans la demande de brevet EP 369 775. L'une diffère de la LAT uniquement par le fait que le promoteur est composé de deux oligonucléotides distincts.

L'autre utilise deux activités enzymatiques, une ligase et une ARN polymérase reconnaissant un promoteur ADN double brin, capable d'élonger sur matrice ARN. Le promoteur, appelé promoteur mobile, est constitué de deux oligonucléotides. L'un porte une séquence promoteur sens et une séquence sonde permettant l'hybridation sur l'extrémité 3' d'une cible ARN, l'autre porte seulement une séquence promoteur anti-sens. Par hybridation, l'extrémité 5' de l'oligonucléotide promoteur antisens est juxtaposée à l'extrémité 3' de la cible, puis ligaturée avec cette même extrémité. La transcription par l'ARN polymérase appropriée résulte dans la synthèse de multiples transcrits. Ces transcrits sont hybridés et ligaturés à un second promoteur mobile. La transcription de la matrice ARN résulte dans la synthèse de transcrits complémentaires. Le processus peut être réitéré, aboutissant à une amplification exponentielle de la séquence cible initiale.

On peut également citer la méthode décrite dans la demande de brevet WO 99/4385 qui est une méthode d'amplification aspécifique des acides nucléiques. Cette méthode d'amplification est également fondée sur la transcription mais ne nécessite qu'une seule amorce. Celle-ci, bloquée en 3', permet l'ajout d'une séquence promotrice T7 en 3' d'un ARN sens. Cette séquence permet alors la transcription d'ARNc antisens. Cette méthode est non spécifique car l'amorce utilisée vient s'hybrider sur la queue polyA des ARNm.

D'une manière générale, les méthodes d'amplification telles que la PCR ou la NASBA nécessitent deux amorces pour l'obtention d'un amplicon. Il en résulte alors que les produits de la réaction d'amplification deviennent eux-mêmes des matrices pour l'amplification, rendant l'amplification de type exponentiel. Si ces méthodes permettent d'obtenir de très bons rendement d'amplification, elles restent mal adaptées pour l'amplification simultanée de plusieurs cibles (on parle d'amplification multiplexe), étape pourtant indispensable lorsqu'on veut analyser un profil d'expression de plusieurs gènes. De plus, la méthode décrite dans la demande WO 99/4385 repose sur l'amplification de l'ensemble des ARNm, ce qui peut contribuer à produire du bruit de fond lors d'une analyse sur puce à ADN. De plus, cette méthode est limitée à la région 3' des gènes. Cette méthode est donc mal adaptée pour l'étude d'une mutation ou d'une région cible situées au milieu du gène ou dans sa région 5'.

La présente invention se propose de résoudre l'ensemble des inconvénients de l'état de la technique en présentant une méthode d'amplification, spécifique, linéaire, qui permet d'amplifier simultanément des régions cibles de gènes différents, lesdites régions cibles pouvant être localisé en 3', 5', ou au milieu du gène.

A ce titre, l'invention concerne une méthode pour générer des transcrits à partir
- d'une séquence ARN à amplifier comprenant une région « amorce » et une région d'intérêt et
- d'une amorce d'amplification comprenant une région promotrice, et une région capable de s'hybrider sur ladite région « amorce » de la séquence ARN à amplifier,
ladite méthode étant réalisée à température constante, et comprenant les étapes suivantes :
a. on hybride ladite amorce avec l'ARN à amplifier,
b. on élonge l'amorce par une activité enzymatique de transcriptase inverse afin de générer une séquence d'acide désoxyribonucléique complémentaire (ADNc) de l'ARN à amplifier,
c. on clive l'ARN à amplifier, hybridé sur ledit ADNc, par une enzyme ayant une activité ribonucléase H, pour obtenir des fragments d'ARN à amplifier, hybridés sur ledit ADNc,
d. on élonge les extrémités desdits fragments d'ARN à amplifier par une enzyme de transcritpase inverse et de déplacement de brin, pour obtenir des hybrides ARN-ADN / ADN
e. on obtient des transcrits d'ARN à partir des hybrides ARN-ADN / ADN formés lors de l'étape d) par une enzyme ayant une activité ARN polymerase.

Au sens de la présente invention, on entend par transcrit un produit de transcription, c'est-à-dire un ARN néosynthétisé lors de la transcription de la matrice sous la dépendance du promoteur ayant initié la transcription.

On entend par séquence, une séquence nucléotidique contenant les bases naturelles (A, C, G, T s'il s'agit d'une séquence de nature desoxyribonucléotidique, A, C, G, U s'il s'agit d'une séquence de nature ribonucléotidique) naturelle et/ou une ou plusieurs bases modifiées, telles que l'inosine, la méthyl-5-desoxycytidine, la désoxyuridine, la diméthylamino-5-désoxyuridine, la diamino-2,6-purine, la bromo-5-désoxyuridine ou toutes bases modifiées, permettant une réaction d'hybridation. La séquence polynucléotidique peut aussi être modifiée au niveau des liaisons internucléotidiques (pouvant impliquer par exemple des liaisons phosphorothioate, Hphosphonate, alkyl phosphonate), ou au niveau du squelette comme c'est le cas par exemple pour les alpha-oligonucléotides (brevet français N° 2 607 507) ou les PNA (EGHOLM et al., 1992. J. Am. Chem. Soc. 1141895-1897). Dans une séquence polynucléotidique modifiée, plusieurs modifications telles qu'indiquées ci-dessus peuvent être présentes en combinaison. Cette séquence peut être de nature ADN, ARN ou de nature chimère ADN/ARN et possède géneralemart une "longueur" d'au moins 5 désoxyribonucléotides et/ou ribonucléotides, pouvant contenir éventuellement au moins un nucléotide modifié. Une séquence peut contenir différentes régions, chacune associées à différentes fonctions.

Pour mettre en oeuvre la méthode selon l'invention, on part d'une séquence ARN à amplifier, qui pourrait être qualifié de premier modèle d'ARN à amplifier. Au sens de la présente invention, l'ARN à amplifier peut être une séquence quelconque d'acide ribonucléique. Préférentiellement, l'ARN à amplifier est un ARN messager.

L'ARN à amplifier comprend notamment une région d'intérêt, c'est à dire un polynucléotide suceptible d'être hybridée par une sonde d'hybridation, spécifique de ladite région d'intérêt. Cette sonde permet ainsi, telle que décrit ci après, la détection de la présence ou de l'absence de la région d'intérêt.

Cet ARN à amplifier comprend également une région « amorce », c'est à dire un polynucléotide sur lequel peut venir s'hybrider spécifiquement une amorce d'amplification telle que définie ci après. Préférentiellement, la région « amorce » est située dans la région 3' de la région d'intérêt de la séquence ARN à amplifier.

Par séquence ou région capable de s'hybrider sur une autre séquence/région, on entend une séquence ou une région pouvant s'hybrider sur une autre séquence/région dans des conditions d'hybridation, qui peuvent être déterminées dans chaque cas de façon connue. On parle également de séquences/régions complémentaires. Une séquence ou région strictement complémentaire d'une autre est une séquence dans laquelle chacune des bases peut s'apparier avec une base de l'autre séquence, sans mésappariement. Par hybridation, on entend le processus au cours duquel, dans des conditions appropriées, deux fragments nucléotidiques, ayant des séquences suffisamment complémentaires sont susceptibles de former un double brin avec des liaisons hydrogènes stables et spécifiques. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques.

La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier.

Au sens de la présente invention, on entend par amorce d'amplification une séquence qui, lorsqu'elle est hybridée sur un acide nucléique (de nature ADN ou ARN) à amplifier, permet d'amorcer une réaction d'élongation. L'amorce utilisée dans la présente invention peut être divisée en 2 régions, qui sont, dans le sens 5' - 3' :
- une région promotrice, suceptible d'être reconnue par une ARN polymérase pour initier la transcription, c'est à dire la synthèse d'un ARN. Cette région promotrice comprend un promoteur et un site d'initiation de la transcrition par une ARN polymérase. Cette région promotrice comprend notamment un des brins d'un promoteur d'une ARN polymérase. A titre d'exemple, on peut citer les promoteurs naturels des ARN polymérases, des séquences raccourcies dérivées des promoteurs naturels et ayant conservé leur fonctionnalité, ou encore les structures "boucles" capables d'initier la transcription telles que décrites dans MOLLEGAARD N.E. et al. 1994, Proc. Natl. Acad. Sci. USA ,91, 3892-3895. Cette région promotrice peut comprendre également une séquence favorable à l'initiation de la transcription, dont la fonction est de limiter la formation de transcrits abortifs. L'ARN polymérase peut être ADN dépendante, ce qui permet la transcription d'un ARN à partir d'une séquence d'ADN.
- une région capable de s'hybrider sur la région « amorce » de l'ARN à amplifier, c'est à dire complémentaire de ladite région «amorce » de l'ARN à amplifier. Cette région peut être de nature ADN.

Ainsi, l'extrémité 5' de l'amorce peut comprendre des nucléotides ne s'hybridant pas avec ledit ARN à amplifier (séquence antisense d'un promoteur, séquence antisense d'un site d'initiation de transcription...), alors que l'extrémité 3 doit avoir une longueur et une composition en nucléotides permettant l'initiation de l'élongation de l'amorce, dans des conditions de température et de stringence appropriées. Selon un mode préféré de réalisation de l'invention, l'amorce est de nature ADN. L'amorce n'est pas bloqué en 3', ce qui permet de stabiliser l'hybride ADN-ARN.

La méthode selon l'invention est une méthode isotherme, c'est à dire qu'elle ne nécessite pas de changement de température au cours des différentes étapes. Selon un mode préféré de réalisation de l'invention, la méthode est réalisée à une température comprise entre 37°C et 50°C, préférentiellement entre 40 et 45°C, et encore plus préférentiellement à 41°C.
Lors de l'étape a), on hybride ladite amorce avec l'ARN à amplifier au niveau de ladite région permettant l'hybridation amorce / séquence ARN à amplifier. Cette hybridation s'effectue par complémentarité d'une région prédéterminée de l'ARN à amplifier avec une région prédéterminé de l'amorce, dans des conditions de stringence et de température appropriée. Selon un mode préféré de réalisation de l'invention, cette étape est précédée d'une étape de dénaturation de l'ARN à amplifier, préférentiellement à 65°C.
Lors de l'étape b), on élonge l'amorce par une activité enzymatique de transcriptase inverse (appelée également reverse transcriptase) afin de générer une séquence d'acide désoxyribonucléique complémentaire (ADNc) de l'ARN à amplifier. Cette étape nécessite la présence de désoxyribonucléotides, qui sont utilisés par l'enzyme pour la synthèse par complémentarité de l'ADNc. Cette élongation s'effectue dans le sens 5' - 3' de l'amoce, dans des conditions de stringence et de température appropriée, comparables à celles de l'étape a). Cette enzyme de transcriptase inverse est préférentiellement l'AMV reverse transcriptase.
Lors de l'étape c), on clive l'ARN hybridé sur ledit ADNc par une enzyme ayant une activité RNase H, pour obtenir des fragments d'ARN hybridés sur ledit ADNc. L'activité RNAse H permet l'hydrolyse les hybrides ARN - ADN, alors qu'elle n'hydrolyse pas les hybrides ARN - ARN ou ADN - ADN. L'activité RNAse H peut être obtenue par une enzyme séparée, telle que notamment par l'enzyme RNAse H dE. coli ou via l'activité RNAseH d'une enzyme reverse transcriptase, telle que notamment l'AMV reverse transcriptase.
Lors de l'étape d), on élonge les extrémités desdits fragments d'ARN par une enzyme de transcriptase inverse et de déplacement de brin, pour obtenir des complexes ARN-ADN / ADN. Cette enzyme de transcriptase inverse est préférentiellement l'AMV reverse transcriptase.
Lors de l'étape e), on obtient des transcrits d'ARN à partir des complexes ARN-ADN / ADN formés lors de l'étape d) par une enzyme ayant une activité ARN polymerase. La transcription s'effectue par synthèse, dans le sens 5' - 3', d'un ARN anti-parralèlle et complémentaire du brin nucléotidique transcrit.

L'enzyme ayant une activité ARN polymerase qui est utilisée est une enzyme capable de se lier au promoteur de l'amorce, et d'initier in vitro la synthèse d'ARN à partir du site d'initiation de la transcription de l'amorce. Selon un mode préféré de réalisation de l'invention, l'enzyme ayant une activité ARN polymérase est une ARN polymerase de bacteriophage, préférentiellement l'ARN polymérase du bactériophage T7, mais d'autres polymérases peuvent être utilisées telles que les ARN polymerase des bactériophages T3, SP6, gh-1... Bien évidement, la séquence promoteur située sur l'amorce doit être adaptée à l'ARN polymérase utilisée lors de l'étape e).

La méthode d'amplification selon l'invention peut se dérouler par l'addition, dans des conditions appropriées, de tous les composés nécessaires à sa mise en oeuvre, c'est à dire l'ARN à amplifier, les enzymes, l'amorce d'amplification, les desoxyribonucléotides. Ce milieu réactionnel est dépourvu d'agents suceptibles d'interferer avec le processus d'amplification, tels que les substances qui pourraient inhiber l'activité des enzymes nécessaires, qui pourraient interferer avec l'hybridation de l'amorce sur l'ARN a amplifier, ou qui pourraient dégrader les produits amplifiés.

Ainsi, selon un mode préféré de réalisation de l'invention, on effectue les étapes a) à e) pendant un temps suffisant pour obtenir un nombre suffisant de transcrits. Préférentiellement, on effectue les étapes a) à e) pendant une durée comprise entre 30 min et 3h30, et encore plus préférentiellement entre 1h30 et 2h30.

Les transcrits ainsi obtenus peuvent ensuite être détectés par toutes les techniques connues de l'homme du métier.

Selon un mode préféré de réalisation de l'invention, la méthode pour générer des transcrits selon l'invention comprend en outre l'étape suivante :
f. on utilise une sonde d'hybridation spécifique de ladite région d'intérêt pour détecter les transcrits d'ARN générés.

Par détection, on entend soit une détection directe par une méthode physique, soit une méthode de détection à l'aide d'un marqueur. De nombreuses méthodes de détection existent pour la détection des acides nucléiques. [Voir par exemple Kricka et al., Clinical Chemistry, 1999, n° 45(4), p.453-458 ou Keller G.H. et al., DNA Probes, 2nd Ed., Stockton Press, 1993, sections 5 et 6, p.173-249]. Par marqueur, on entend un traceur capable d'engendrer un signal. Une liste non limitative de ces traceurs comprend les enzymes qui produisent un signal détectable par exemple par colorimétrie, fluorescence ou luminescence, comme la peroxydase de raifort, la phosphatase alcaline, la bêtagalactosidase, la glucose-6-phosphate déshydrogénase ; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I. Ainsi, le polynucléotide peut être marqué pendant l'étape d'amplification selon l'invention, par exemple en utilisant un nucléotide triphosphate marqué pour la réaction d'amplification. Des systèmes indirects peuvent aussi être utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticoips, sucre/lectine, polynucléotide/complémentaire du polynucléotide, séquence successive d'histidines, dite « tag », pour un métal, par exemple le nickel. Dans ce cas, c'est le ligand qui porte l'agent de liaison. L'anti-ligand peut être détectable directement par les marqueurs décrits au paragraphe précédent ou être lui-même détectable par un ligand/anti-ligand.

Le nucléotide marqué sera un ribonucléotide. Le polynucléotide peut aussi être marqué après l'étape d'amplification, par exemple en hybridant une sonde marquée selon la technique d'hybridation sandwich décrite dans le document WO-A-91/19812. Des telles sondes peuvent être également utilisées dans une technique OLISA, telle que décrite dans la demande WO 03/098217.

Cette détection peut être également mis en oeuvre par l'utilisation de sondes d'hybridation qui reconnaissent lesdites régions d'intérêt des ARN amplifiés selon l'invention. Par sonde d'hybridation, on entend un fragment nucléotidique comprenant de 5 à 100 motifs nucléotidiques, notamment de 6 à 35 motifs nucléotidiques, possédant une spécificité d'hybridation dans des conditions déterminées pour former un complexe d'hybridation avec une région d'intérêt. Cette sonde d'hybridation peut être une sonde dite de capture. Dans ce cas, le fragment nucléotidique cible peut être préalablement marqué au moyen d'un marqueur. La sonde dite de capture est immobilisée ou immobilisable sur un support solide par tout moyen approprié, c'est-à-dire directement ou indirectement, par exemple par covalence ou adsorption. On réalise alors une réaction d'hybridation entre ladite sonde et le fragment nucléotidique cible marqué.

La sonde d'hybridation peut également être une sonde dite de détection. Dans ce cas, la sonde d'hybridation peut être marquée au moyen d'un marqueur .On réalise alors une réaction d'hybridation entre ladite sonde de détection et le fragment nucléotidique cible. Un test sandwich peut également mettre en oeuvre une sonde de capture fixée sur un support solide, sur laquelle s'hybride le fragment nucléotide cible, sur lequel s'hybride la sonde de détection.

Que l'on utilise une sonde dite de capture ou une sonde dite de détection, la réaction d'hybridation peut être réalisée sur un support solide qui inclut tous les matériaux sur lesquels peut être immobilisé un acide nucléique. Des matériaux de synthèse ou des matériaux naturels, éventuellement modifiés chimiquement, peuvent être utilisés comme support solide, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO-A-94/12670, d'une particule ou d'une biopuce. Par biopuce, on entend un support solide de dimension réduite où sont fixée une multitude de sondes de capture à des positions prédéterminées. Le concept de biopuce, plus précisément de puce à ADN, date du début des années 90. De nos jours, ce concept s'est élargi puisque des puces à protéines commencent à se développer. Il repose sur une technologie pluridisciplinaire intégrant la micro-électronique, la chimie des acides nucléiques, l'analyse d'images et l'informatique. Le principe de fonctionnement repose sur un fondement de la biologie moléculaire: le phénomène d'hybridation, c'est-à-dire l'appariement par complémentarité des bases de deux séquences d'ADN et/ou d'ARN.

La méthode des biopuces repose sur l'emploi de sondes de capture fixées sur un support solide sur lesquelles on fait agir un échantillon de fragments nucléotidiques cibles marqués directement ou indirectement avec des fluorochromes. Les sondes de capture sont positionnées de manière spécifique sur le support ou puce et chaque hybridation donne une information particulière, en relation avec le fragment nucléotidique cible. Les informations obtenues sont cumulatives, et permettent par exemple de quantifier le niveau d'expression d'un gène ou de plusieurs gènes cibles. Pour analyser l'expression d'un gène cible, on peut alors réaliser une biopuce portant de très nombreuses sondes qui correspondent à tout ou partie du gène cible, qui est transcrit en ARNm. On hybride alors par exemple les ADN complémentaires des ARNm issues du ou des gènes cibles que l'on souhaite analyser. Après hybridation, le support ou puce est lavé(e), lu(e) par exemple par un scanner et l'analyse de la fluorescence est traitée par informatique. On peut citer à titre indicatif, les puces à ADN mises au point par la société Affymetrix ("Accessing Genetic Information with High-Density DNA arrays", M. Chee et al., Science, 1996, 274, 610-614. "Light-generated oligonucleotide arrays for rapide DNA sequence analysis", A. Caviani Pease et al., Proc. Natl. Acad. Sci. USA, 1994, 91, 5022-5026), pour les diagnostics moléculaires. Dans cette technologie, les sondes de capture sont généralement de tailles réduites, autour de vingt nucléotides. D'autres exemples de biopuces sont donnés dans les publications de G. Ramsay, Nature Biotechnology, 1998, n°16, p. 40-44 ; F. Ginot, Human Mutation, 1997, n°10, p.1-10 ; J. Cheng et al, Molecular diagnosis, 1996, n°1(3), p.183-200 ; T. Livache et al, Nucleic Acids Research, 1994, n° 22(15), p. 2915-2921 ; J. Cheng et al, Nature Biotechnology, 1998, n° 16, p. 541-546 ou dans les brevets US-A-4,981,783, US-A-5,700,637, US-A-5,445,934, US-A-5,744,305 et US-A-5,807,522. On peut également citer les puces basses densités développées par la société Apibio telles que décrites dans la demande WO 03/098217. La caractéristique principale du support solide doit être de conserver les caractéristiques d'hybridation des sondes de capture sur les fragments nucléotidiques cibles tout en générant un bruit de fond minimum pour la méthode de détection. La présente invention est particulièrement adaptée à ces systèmes de puces basse densité.

La méthode selon l'invention peut être mise en oeuvre pour générer des transcrits d'ARN provenant de différents gènes. C'est même un des avantages de la présente invention qui permet d'amplifier simultanément des régions cibles de gènes différents, lesdites régions cibles pouvant être localisé en 3', 5', ou au milieu du gène.

A ce titre, l'invention concerne également une méthode pour générer des transcrits à partir
- d'au moins une première séquence ARN à amplifier comprenant une région « amorce » et une région d'intérêt et d'une première amorce d'amplification comprenant une région promotrice, et une région capable de s'hybrider sur ladite région « amorce » de la première séquence ARN à amplifier,
- d'au moins une deuxième séquence ARN à amplifier, différente de ladite première séquence ARN à amplifier, et comprenant une région «amorce » et une région d'intérêt et d'une deuxième amorce d'amplification comprenant une région promotrice, et une région capable de s'hybrider sur ladite région «amorce » de la deuxième séquence ARN à amplifier,
ladite méthode étant réalisée à température constante, et comprenant les étapes suivantes :
a). on hybride lesdites premières et deuxièmes amorces avec lesdits premier et deuxième ARN à amplifier,
b). on élonge lesdites premières et deuxièmes amorces par une activité enzymatique de transcriptase inverse afin de générer une première séquence d'acide désoxyribonucléique complémentaire (ADNc) du premier ARN à amplifier et une deuxième séquence d'acide désoxyribonucléique complémentaire (ADNc) du deuxième ARN à amplifier,
c). on clive les premiers et deuxième ARN à amplifier, hybridé respectivement sur le premier et le deuxième ADNc, par une enzyme ayant une activité ribonucléase H, pour obtenir des premiers fragments d'ARN à amplifier, hybridés sur le premier ADNc, et des deuxième fragments d'ARN à amplifier, hybridés sur le deuxième ADNc
d). on élonge les extrémités desdits premiers et deuxièmes fragments d'ARN à amplifier par une enzyme de transcritpase inverse et de déplacement de brin, pour obtenir des hybrides ARN-ADN / ADN
e). on obtient des transcrits desdits premiers et deuxième ARN à amplifier, à partir des hybrides ARN-ADN / ADN formés lors de l'étape d) par une enzyme ayant une activité ARN polymerase.

Cette méthode peut être appliquée d'une manière comparable pour genérer des transcrits à partir d'ARN à amplifier provenant de 3, 4, 5, 10, ou plus gènes différents. Les étapes a) à e) sont effectuées comparablement à celles décrites précédemment, et sont réalisées pendant un temps suffisant pour obtenir un nombre suffisant de transcrits, comme défini précédemment.

Pour la détection des transcrits, on utilise préférentiellement, lors d'une étape f), une première sonde d'hybridation pour détecter les transcrits du premier ARN à amplifier et une deuxième sonde d'hybridation pour détecter les transcrits du deuxième ARN à amplifier.

Préférentiellement, et comme défini précédemment, l'enzyme ayant une activité ARN polymérase est une ARN polymerase de bacteriophage, et encore plus préférentiellement l'ARN polymérase du bactériophage T7.

Les figures ci-jointes sont données à titre d'exemple explicatif et n'ont aucun caractère limitatif. Elles permettront de mieux comprendre l'invention.
La figure 1 représente schématiquement les étapes de la méthode selon l'invention.

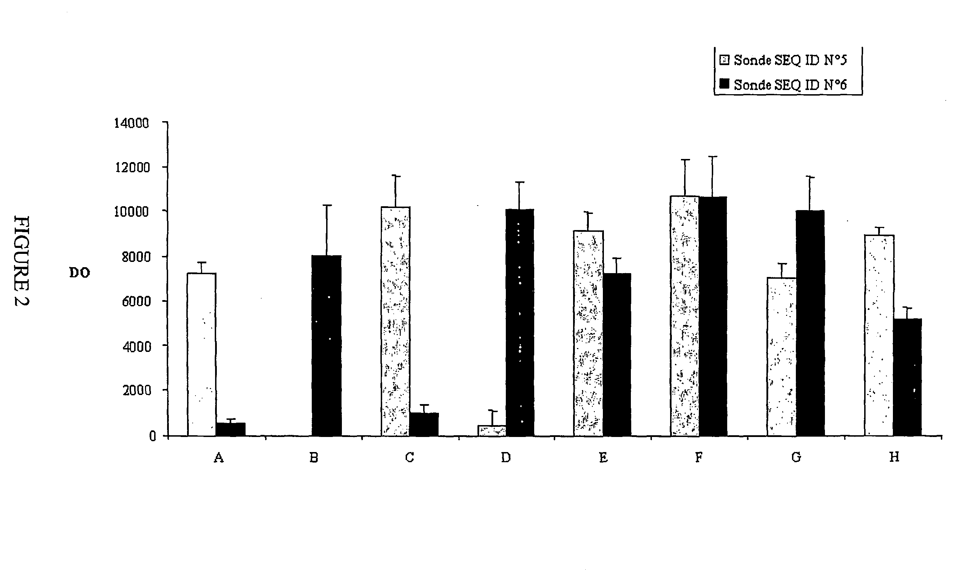

La figure 2 représente le signal émis par une sonde d'hybridation hybridée sur des transcrits obtenus selon l'invention et tel que décrit dans l'exemple 2.
La figure 3 représente la régression linéaire obtenues après amplification d'une gamme de transcrits (1.10⁷ , 1.10⁸ , 1.10⁹ et 1.10¹⁰ copies) selon le protocole décrit dans l'exemple 3.
La figure 4 représente le signal émis (moyenné sur 3 réplicats) et son écart-type au niveau de chaque sonde spécifique après hybridation et révélation des produits d'amplifications obtenus selon le protocole décrit dans l'exemple 4.

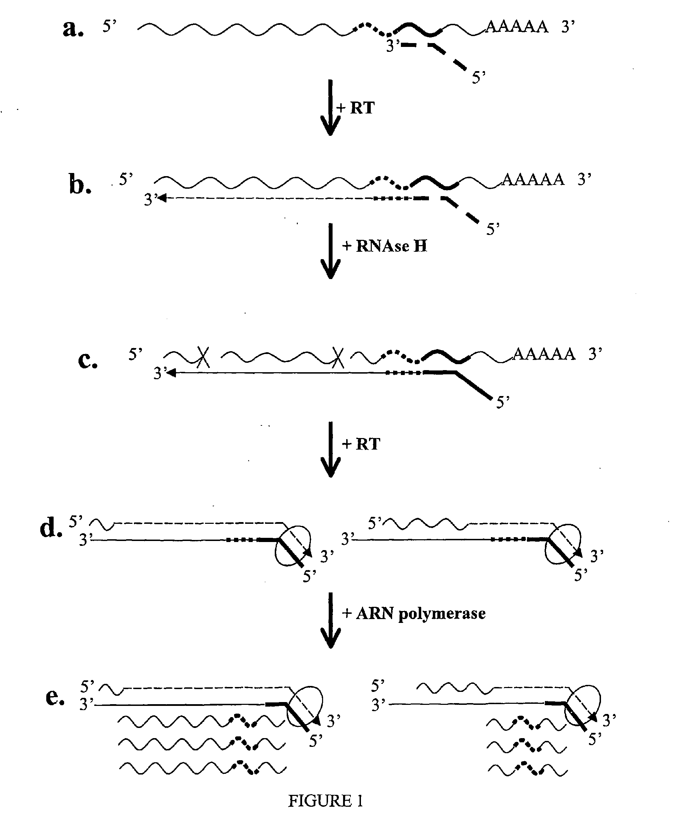

Sur la figure 1, les séquences de nature ribonuléique sont représentées en trait ondulé, alors que les séquences de nature desoxyribonucléique sont représentées en trait droit.

Lors de l'étape a), on hybride l'amorce de nature ADN (représentée par un trait en long pointillé) sur la région «amorce » (représentée par un trait gras) de l'ARN matrice, la séquence promotrice en 5' de l'amorce ne s'hybridant pas sur l'ARN matrice. Pour mieux comprendre l'invention, la séquence d'intérêt que l'on souhaite détecter après la phase d'amplification est représenté en pointillé court, du coté 5' de la région « amorce ». Toutefois, la séquence d'interêt et la séquence « amorce » peuvent être une seule et même séquence.

On fait agir, lors de l'étape b) une enzyme ayant une activité de reverse transcriptase (RT), afin d'élonger, dans le sens 5' - 3', l'amorce hybridée sur l'ARN matrice. On obtient un ADN (ADNc, représenté en trait pointillé) dont la séquence est complémentaire à celle de l'ARN matrice. On obtient ainsi, entre la région cible et l'extrémité 5' de l'ARN matrice, une séquence hybride double brin ARN matrice / ADNc. De part leur absence de complémentarité, la séquence promotrice de l'amorce, et la séquence située coté 3' de la région cible de l'ARN cible restent des séquences non hybridés (simple brin).

On fait agir, lors de l'étape c, une enzyme ayant une activité RNAse H pour induire des coupures au sein de l'ARN matrice. Ces coupures sont non spécifiques, et permettent de libérer l'extremité 3' OH (représenté par une croix) de fragments d'ARN, hybridés sur l'ADNc.

Les extremités 3' OH ainsi libérées sont élongés lors de l'étape d) par une enzyme ayant une activité reverse transcriptase et une activité de déplacement de brin. L'élongation se termine par la synthèse du brin complémentaire de la séquence promotrice de l'amorce. On obtient ainsi différentes séquences hybrides double brin ARN-ADN / ADN, comprenant une région promotrice double brin (représenté par un cercle), capable, sous l'action d'une ARN polymérase ADN dépendant, d'initier une transcription. Deux hybrides différents, obtenus à partir de deux sites de coupure différents sont ainsi représentés à titre indicatif.

L'action d'une enzyme ARN polymérase ADN dépendante permet d'obtenir, lors de l'étape e) des transcrits d'ARN, correspondants aux différents hybrides obtenus lors de l'étape d). On obtient ainsi une population de transcrits, de taille différente mais comprenant tous la région d'intérêt que l'on souhaite détecter.

Les exemples suivants sont donnés à titre illustratif et n'ont aucun caractère limitatif. Ils permettront de mieux comprendre l'invention.

### Exemple 1 : obtention de séquences ARN à amplifier

Une première séquence d'ARN à amplifier, correspondant à la SEQ ID N°1 : a été produite à partir de séquences clonées issues d'une région du gène *pol* du rétrovirus endogène HML-4 dans un vecteur Dual promoter pCR-II-TOPO (Invitrogen) selon le protocole recommandé par le fournisseur. La transcription a été réalisée à l'aide du Kit Ampliscribe T7 Flash (Epicentre) selon le protocole recommandé par le fournisseur.

D'une manière comparable, une deuxième séquence d'ARN à amplifier, de SEQ ID N°2 : a été produite à partir de séquences clonées issues d'une région du gène *pol* du rétrovirus endogène HERV-E4.1.

### Exemple 2 : Génération de transcrits par la méthode selon l'invention à partir de séquences d'ARN telles que définies dans l'exemple 1

Pour l'amplification de la séquence ARN de SEQ ID N°1, on utilisait une séquence amorce de SEQ ID N°3 :
SEQ ID N°3: *TAATACGACT CACTATA***GGG AGA**TCTAGCA GCATAGGGGG GGCTGTGCAG AGGAGATCAT CC.
comprenant
- une région promotrice comprenant un promoteur de la T7 ARN polymérase (indiqué en italique sur la SEQ ID N°3) et un site d'initiation de la transcription (indiqué en gras sur la SEQ ID N°3)
- une région capable de s'hybrider sur une région amorce de la séquence ARN à amplifier (indiqué en souligné sur la SEQ ID N°3)

Cette amorce était non bloquée en 3'.

A noter que la région capable de s'hybrider sur la région amorce peut être plus courte, et comprendre notamment de 15 à 30 paires de bases. A noter également que entre le site d'initiation et la région capable de s'hybrider sur la région amorce, est comprise une séquence favorisant l'initiation, qui limite la formation de transcrits abortifs.

Pour l'amplification de la séquence ARN de SEQ ID N°2, on utilisait une séquence amorce de SEQ ID N° 4
SEQ ID N° 4: *TAATACGACT CACTATA****GGG* AGA**TCTAGCA GCATACAGCA AAAGGTCATC AACGTACTGG AG
comprenant
- une région promotrice comprenant un promoteur de la T7 ARN polymérase (indiqué en italique sur la SEQ ID N°4) et un site d'initiation de la transcription (indiqué en gras sur la SEQ ID N°4)
- une région capable de s'hybrider sur une région amorce de la séquence ARN à amplifier (indiqué en souligné sur la SEQ ID N°4)

Cette amorce était non bloquée en 3'.

A noter que la région capable de s'hybrider sur la région amorce peut être plus courte, et comprendre notamment de 15 à 30 paires de bases. A noter également que entre le site d'initiation et la région capable de s'hybrider sur la région amorce, est comprise une séquence favorisant l'initiation, qui limite la formation de transcrits abortifs.

La génération des transcrits était réalisé dans un mélange réactionnel (NucliSens Basic Kit bioMérieux) comprenant une enzyme ayant une activité de reverse transcriptase, (AMV-RT), une enzyme ayant une activité RNaseH (RNase H) et une enzyme ayant une activité ARN polymerase (T7 ARN polymérase).

L'amplification était réalisée en présence :
- la séquence amorce de SEQ ID N° 3 (0,5µM) pour amplifier la séquence d'ARN de SEQ ID N°1,
- la séquence amorce de SEQ ID N° 4 (0,5µM), pour amplifier la séquence d'ARN de SEQ ID N°2,
- la séquence amorce de SEQ ID N° 3 (0,5µM) et la séquence amorce de SEQ ID N°4 (0,5µM) pour amplifier simultanément la séquence d'ARN de SEQ ID N°1 et la séquence d'ARN de SEQ ID N°2.

On réalisait ainsi l'amplification des séquences d'ARN SEQ ID N° 1 et SEQ ID N°2, avec incorporation de biotine :
- indépendemment l'une de l'autre :
   Pour cela, une quantité connue d'ARN de SEQ ID N°1 ou d'ARN de SEQ ID N°2 en solution aqueuse (1.10⁹ ou 1.10'° copies dans 5µL H₂O) est mélangée à lOpL de mélange réactionnel (NucliSens Basic Kit) auquel a été préalablement rajouté l'UTP-biotine (0,6mM) et l'amorce permettant l'amplification de la SEQ ID N°1 ou de la SEQ ID N°2.
- simultanément:
   Pour cela, une quantité connue d'ARN de SEQ ID N°1 et d'ARN de SEQ ID N°2 en solution aqueuse ont été mélangées à lOpL de mélange réactionnel (NucliSens Basic Kit) auquel a été préalablement rajouté l'UTP-biotine et les amorces permettant l'amplification de la SEQ ID N°1 et de la SEQ ID N°2.

Pour chacune des réactions d'amplification, le volume résultant était de 15 µL (qsp H2O nucléase-free). Le mélange était ensuite incubé 5min à 65°C, puis 5 min à 41°C afin de permettre l'hybridation de l'amorce sur l'ARN à amplifier.

Un volume de 5µL de mélange enzymatique (NucliSens Basic Kit) était ensuite ajouté. Le mélange d'un volume de 20 µL était incubé 90min à 41 °C pour permettre
1. l'hybridation de l'amorce avec l'ARN à amplifier,
2. l'élongation de l'amorce par l'activité enzymatique de transcriptase inverse afin de générer une séquence d'acide désoxyribonucléique complémentaire (ADNc) de l'ARN à amplifier,
3. le clivage de l'ARN à amplifier, hybridé sur ledit ADNc, par l'enzyme ayant l'activité ribonucléase H, pour obtenir des fragments d'ARN à amplifier, hybridés sur ledit ADNc,
4. l'élongation des extrémités desdits fragments d'ARN à amplifier par l'enzyme de transcriptase inverse et de déplacement de brin, pour obtenir des hybrides ARN-ADN / ADN
5. l'obtention des transcrits d'ARN à partir des hybrides ARN-ADN / ADN formés lors de l'étape d) par l'enzyme ayant une activité ARN polymerase.

Les transcrits d'ARN obtenus ont été analysés par la méthode ELOSA (Mallet F. et coll. 1993). Cette méthode permet la détection spécifique de séquences d'acides nucléiques par l'hybridation de ceux-ci sur des sondes oligonucléotidiques (appelées sondes de détection) greffées en fond de puits de plaques de microtitration.
La sonde permettant la mise en évidence de la SEQ ID N° 1 était la séquence SEQ ID N°5: CAACCTGTCAGGGATCAGTTTC
La sonde permettant la mise en évidence de la SEQ ID N° 2 était la séquence SEQ ID N°6:CTCGAGACCTCCAGAAGTTTCC
Le marquage des transcrits d'ARN par la biotine permettait la révélation de l'hybridation grâce à l'action d'un conjugué streptavidine-enzyme et du substrat correspondant, et les DO étaient lus par spectrométrie. La DO était proportionnelle à la quantité de transcrits générés par la méthode selon l'invention.

Les résultats obtenus sont présentés dans la figure 1. Les lettres A à D correspondaient à des conditions d'amplification des SEQ ID N°1 et SEQ m N°2 indépendamment l'une de l'autre (A:1.10⁹ copies de SEQ ID N°1 ; B:1.10⁹ copies de SEQ ID N°2 ; C:1.10¹⁰ copies de SEQ ID N°1 ; D:1.10¹⁰ copies de SEQ ID N°2), alors que les lettres E à H correspondaient à des amplifications des SEQ ID N°1 et SEQ ID N°2 simultanément (E:1.10⁹ copies de SEQ ID N°1 et 1.10⁹ copies de SEQ ID N°2 ; F: 1.10¹⁰ copies de SEQ ID N°1 et 1.10¹⁰ copies de SEQ ID N°2 ; G: 1.10⁹ copies de SEQ ID N°1 et 1.10¹⁰ copies de SEQ ID N°2 ; H: 1.10¹⁰ copies de SEQ ID N°1 et 1.10⁹ copies de SEQ ID N°2). Les transcrits obtenus étaient détectés par la présence d'une sonde de détection de SEQ ID N°5 (colonnes grises) ou par la présence d'une sonde de détection de SEQ ID N°6 (colonnes noires).

Les résultats montraient que les signaux détectés étaient comparables entre les amplifications indépendantes et les amplifications réalisées en duplexes.
Ainsi, le nombre de transcrits générés étaient comparables que l'ARN à amplifier ait une SEQ ID N°1 ou une SEQ ID N°2 (la DO lue était comparable lors de l'amplification selon la condition A et la condition B). De plus, le nombre de transcrits générés étaient plus important lorsque la quantité d'ARN à amplifier était augmenté (la DO lue était inférieure lors de l'amplification selon la condition A que selon la condition C ; de même, la DO lue était inférieure lors de l'amplification selon la condition B que selon la condition D).
Enfin, le nombre de transcrits générés lors de l'amplification simultané des SEQ ID N°1 et SEQ ID N°2 était comparable au nombre de transcrits générés lors de l'amplification des SEQ ID N°1 et SEQ ID N°2 indépendamment l'une de l'autre. Même lorsque l'amplification simultanée est réalisé sur des quantités de transcrits différant d'un log, les signaux restent identiques à ceux obtenus lors de l'amplification des SEQ ID N°1 et SEQ ID N°2 independamment l'une de l'autre.

Des résultats comparables étaient obtenus par l'analyse des transcrits générés sur puce OLISA. Les puces OLISA sont des puces à ADN basse-densité (jusqu'à 128 sondes par puce) disposées sur un format standard de plaque 96 puits. Les sondes de détection oligonucléotidiques, réparties en couronnes, sont greffées en fond de puits. La révélation colorimétrique des hybrides sonde/cible se fait à l'aide d'un conjugué streptavidine-enzyme qui implique un marquage biotine de la cible.

Une étape supplémentaire de digestion par la RNase confirmait que le produit détecté est bien un cRNA.

Ces résultats démontrent que la méthode selon l'invention est donc particulièrement adaptée à l'amplification de transcrits en vue de leur analyse quantitative sur puce à ADN. En effet, le fait que cette méthode soit transcriptionnelle et que les produits d'amplification ne deviennent jamais des matrices pour l'amplification confère à cette amplification un caractère linéaire parfaitement adapté à une quantification sur biopuce des transcrits.

### Expérience 3 : Evaluation de la linéarité de l'amplification de la méthode selon l'invention

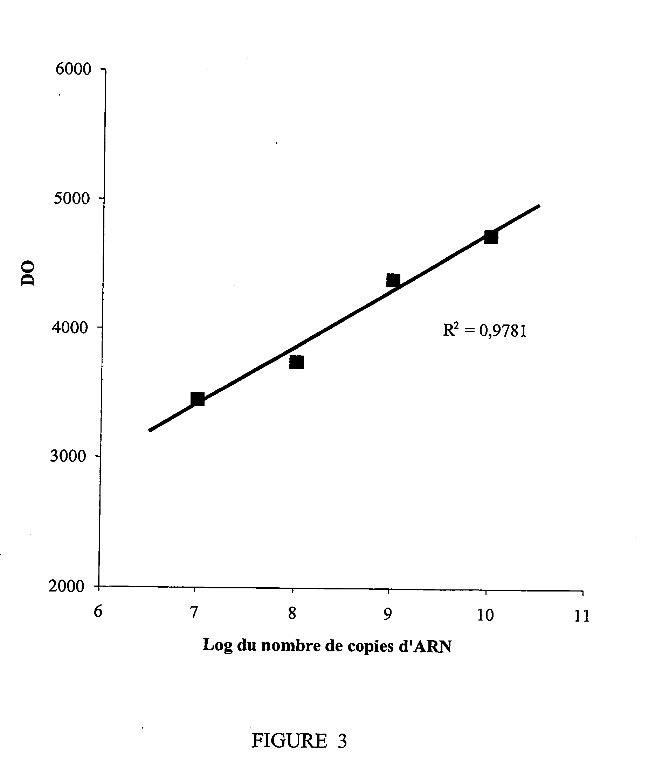

Cet exemple est réalisé à partir de la SEQ ID N°2 utilisée comme séquence ARN à amplifier et la SEQ ID N°4 utilisée en tant qu'amorce d'amplification.
La méthode pour générer les transcrits est réalisée selon les conditions décrites dans l'exemple 2. Dans ce cas, l'amorce de SEQ ID N°2 est utilisée à une concentration de 0,5 µM. Une gamme de transcrits (1.10⁷ , 1.10⁸ , 1.10⁹ et 1.10¹⁰ copies) est amplifiée et les produits de la réaction sont analysés sur puce OLISA à l'aide de la sonde de SEQ ID N°6 , selon un principe comparable à celui développé dans l'exemple 2.
La régression linéaire réalisée à partir des moyennes obtenues pour au moins 4 mesures de chaque point conduit à l'obtention d'une droite de coefficient de corrélation R²=0,9781, telle que présentée dans la figure 3. Ceci atteste donc de la linéarité de l'amplification de la méthode selon l'invention.

### Expérience 4 : Amplification en multiplex

Pour cette expérience, le protocole d'amplification a été réalisé comme précédemment décrit dans les exemples 1 et 2.
Ainsi, une première séquence d'ARN à amplifier, correspondant à la SEQ ID N°7 : a été produite à partir de séquences clonées issues d'une région du gène *pol* du rétrovirus endogène HML-2 dans un vecteur Dual promoter pCR-II-TOPO (Invitrogen) selon le protocole recommandé par le fournisseur. La transcription a été réalisée à l'aide du Kit Ampliscribe 1-7 F7ash (Epicentre) selon le protocole recommandé par le fournisseur.

D'une manière comparable, une deuxième séquence d'ARN à amplifier, de SEQ ID N°8 : a été produite à partir de séquences clonées issues d'une région du gène *pol* du rétrovirus endogène HML-5 dans un vecteur Dual promoter pCR-II-TOPO (Invitrogen) selon le protocole recommandé par le fournisseur. La transcription a été réalisée à l'aide du Kit Ampliscribe T7 Flash (Epicentre) selon le protocole recommandé par le fournisseur.

D'une manière comparable, une troisième séquence d'ARN à amplifier, de SEQ ID N°9 : a été produite à partir de séquences clonées issues d'une région du gène *pol* du rétrovirus endogène HERV-H dans m vecteur Dual promoter pCR-ll- TOPO (Invitrogen) selon le protocole recommandé par le fournisseur. La transcription a été réalisée à l'aide du Kit Ampliscribe T7 Flash (Epicentre) selon le protocole recommandé par le fournisseur.

D'une manière comparable, une quatrième séquence d'ARN à amplifier, de SEQ ID N°10 a été produite à partir de séquences clonées issues d'une région du gène *pol* du rétrovirus endogène ERV-9 dans un vecteur Dual promoter pCR-II-TOPO (Invitrogen) selon le protocole recommandé par le fournisseur. La transcription a été réalisée à l'aide du Kit Ampliscribe T7 Flash (Epicentre) selon le protocole recommandé par le fournisseur.

D'une manière comparable, une cinquième séquence d'ARN à amplifier, de SEQ ID N°11: a été produite à partir de séquences clonées issues d'une région du gène *pol* du rétrovirus endogène HERV-W dans un vecteur Dual promoter pCR-II-TOPO (Invitrogen) selon le protocole recommandé par le fournisseur. La transcription a été réalisée à l'aide du Kit Ampliscribe T7 Flash (Epicentre) selon le protocole recommandé par le fournisseur.

D'une manière comparable, une sixième séquence d'ARN à amplifier, de SEQ ID N°12 : a été produite à partir de séquences clonées issues d'une région du gène *pol* du rétrovirus endogène HERV-R dans un vecteur Dual promoter pCR-II-TOPO (Invitrogen) selon le protocole recommandé par le fournisseur. La transcription a été réalisée à l'aide du Kit Ampliscribe T7 Flash (Epicentre) selon le protocole recommandé par le fournisseur.

D'une manière comparable, une septième séquence d'ARN à amplifier, de SEQ ID N°13 : a été produite à partir de séquences clonées issues d'une région du gène *pol* du rétrovirus endogène HERV-L dans un vecteur Dual promoter pCR-II-TOPO (Invitrogen) selon le protocole recommandé par le fournisseur. La transcription a été réalisée à l'aide du Kit Ampliscribe T7 Flash (Epicentre) selon le protocole recommandé par le fournisseur.

L'amplification était réalisée, comme décrit dans l'exemple 2, en présence :
- la séquence amorce de SEQ ID N° 14 AATTCTAATA CGACTCACTA TAGGGAGACA CATAAAATAT CATCAACATA (0,75µM) pour amplifier la séquence d'ARN de SEQ ID N°7,
- la séquence amorce de SEQ ID N° 15 AATTCTAATA CGACTCACTA TAGGGAGATG CAGAGGAGAT CATCCATGTA (0,75µM), pour amplifier la séquence d'ARN de SEQ ID N° 1
- la séquence amorce de SEQ ID N° 16 AATTCTAATA CGACTCACTA TAGGGAGACT AGTAAAATAT CATCCATAAA (0,75µM) pour amplifier la séquence d'ARN de SEQ ID N°8,
- la séquence amorce de SEQ ID N° 17 AATTCTAATA CGACTCACTA TAGGGAGAAA AGTAGAAGGT CATCAATATA (0,75µM) pour amplifier la séquence d'ARN de SEQ ID N°9,
- la séquence amorce de SEQ ID N° 18 AATTCTAATA CGACTCACTA TAGGGAGAAG TAAATCATCC ACATACTGAA G (0,75µM pour amplifier la séquence d'ARN de SEQ ID N°10,
- la séquence amorce de SEQ ID N° 19 AATTCTAATA CGACTCACTA TAGGGAGAAA GTAAATCATC CACGTACCGA (0,75µM) pour amplifier la séquence d'ARN de SEQ ID N°11,
- la séquence amorce de SEQ ID N° 20 AATTCTAATA CGACTCACTA TAGGGAGACC AGCAAAAGGT CATCAACGTA (0,75µM) pour amplifier la séquence d'ARN de SEQ ID N°2
- la séquence amorce de SEQ ID N° 21 AATTCTAATA CGACTCACTA TAGGGAGACC AAAAGAAGGT TGTCTATGTA (0,75µM) pour amplifier la séquence d'ARN de SEQ ID N° 12,
- la séquence amorce de SEQ ID N° 22 AATTCTAATA CGACTCACTA TAGGGAGATC ARCATAATGY CATCAATGTA (0,75µM) pour amplifier la séquence d'ARN de SEQ ID N° 13.

Les transcrits d'ARN obtenus ont été analysés sur puce OLISA tels que décrits dans l'exemple 2.
La sonde permettant la mise en évidence de la SEQ ID N° 1 était la séquence SEQ ID N° 5 : CAACCTGTCAGGGATCAGTTTC
La sonde permettant la mise en évidence de la SEQ ID N° 2 était la séquence SEQ ID N° 6 : CTCGAGACCTCCAGAAGTTTCC
La sonde permettant la mise en évidence de la SEQ ID N° 7 était la séquence SEQ ID N° 23 : CAACCAGTTA GAGACAAGTT TTCA
La sonde permettant la mise en évidence de la SEQ ID N° 8 était la séquence SEQ ID N° 24 : TTGCTCCCCA GTAGAAAAGA ATT
La sonde permettant la mise en évidence de la SEQ ID N° 9 était la séquence SEQ ID N° 25 : GACAGCCCCC ATTACTTCAG TCAAG
La sonde permettant la mise en évidence de la SEQ ID N° 10 était la séquence SEQ ID N° 26 : CCAAGATCTA GGCCACTTCT CA
La sonde permettant la mise en évidence de la SEQ ID N° 11 était la séquence SEQ ID N° 27 : CATCTATTTG GCCAGGCATT A
La sonde permettant la mise en évidence de la SEQ ID N° 12 était la séquence SEQ ID N° 28 : AGACCTCAAG GCATACACCC
La sonde permettant la mise en évidence de la SEQ ID N° 13 était la séquence SEQ ID N° 29 : GCTITGTGTC ATAATCTTAT TCG

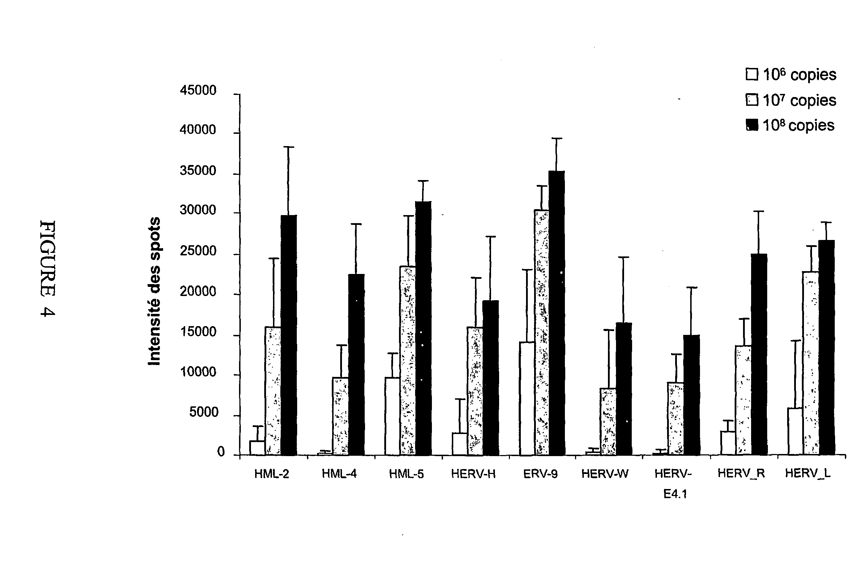

Les moyennes et écart-types sont calculés à partir de quatre amplifications indépendantes, et présentés dans la figure 4.
La technique selon l'invention permet d'amplifier des transcrits en mélange (ici, jusqu'à 9 différents). On constate, de plus, qu'il existe une relation linéaire entre le signal mesuré et la quantité de transcrits introduite dans le volume réactionnel sur la gamme testée (de 10⁶ à 10⁸ copies pour chaque transcrit). Ceci indique que cette technique permet d'amplifier simultanément et de manière spécifique plusieurs transcrits. Ainsi, l'analyse sur puce à ADN (ou par une autre méthode) de produits d'amplification obtenus par cette méthode pourrait permettre une quantification des transcrits de l'échantillon initial.

## Revendications

1. Méthode pour générer des transcrits à partir
• d'au moins une séquence ARN à amplifier comprenant une région «amorce » et une région d'intérêt et
• d'une amorce d'amplification comprenant une région promotrice, et une région capable de s'hybrider sur ladite région « amorce » de la séquence ARN à amplifier,
ladite méthode étant réalisée à température constante, et comprenant les étapes suivantes :
a) on hybride ladite amorce avec l'ARN à amplifier,
b) on élonge l'amorce par une activité enzymatique de transcriptase inverse afin de générer une séquence d'acide désoxyribonucléique complémentaire (ADNc) de l'ARN à amplifier,
c) on clive l'ARN à amplifier, hybridé sur ledit ADNc, par une enzyme ayant une activité ribonucléase H, pour obtenir des fragments d'ARN à amplifier, hybridés sur ledit ADNc,
d) on élonge les extrémités desdits fragments d'ARN à amplifier par une enzyme de transcritpase inverse et de déplacement de brin, pour obtenir des hybrides ARN-ADN / ADN
e) on obtient des transcrits d'ARN à partir des hybrides ARN-ADN / ADN formés lors de l'étape d) par une enzyme ayant une activité ARN polymerase.

2. Méthode pour générer des transcrits selon la revendication 1 comprenant en outre l'étape suivante :
f. on utilise une sonde d'hybridation spécifique de ladite région d'intérêt pour détecter les transcrits d'ARN générés.

3. Méthode pour générer des transcrits selon l'une quelconque des revendications 1 ou 2 selon laquelle on effectue les étapes a) à e) pendant un temps suffisant pour obtenir un nombre suffisant de transcrits.

4. Méthode pour générer des transcrits selon l'une quelconque des revendications 1 à 3 dans laquelle l'enzyme ayant une activité ARN polymérase est une ARN polymerase de bacteriophage, préférentiellement l'ARN polymérase du bactériophage T7.

5. Méthode pour générer des transcrits à partir
• d'au moins une première séquence ARN à amplifier comprenant une région « amorce » et une région d'intérêt et d'une première amorce d'amplification comprenant une région promotrice, et une région capable de s'hybrider sur ladite région « amorce » de la première séquence ARN à amplifier,
• d'au moins une deuxième séquence ARN à amplifier, différente de ladite première séquence ARN à amplifier, et comprenant une région «amorce » et une région d'intérêt et d'une deuxième amorce d'amplification comprenant une région promotrice, et une région capable de s'hybrider sur ladite région « amorce » de la deuxième séquence ARN à amplifier,
ladite méthode étant réalisée à température constante, et comprenant les étapes suivantes:
a). on hybride lesdites premières et deuxièmes amorces avec ledits premier et deuxième ARN à amplifier,
b). on élonge lesdites premières et deuxièmes amorces par une activité enzymatique de transcriptase inverse afin de générer une première séquence d'acide désoxyribonucléique complémentaire (ADNc) du premier ARN à amplifier et une deuxième séquence d'acide désoxyribonucléique complémentaire (ADNc) du deuxième ARN à amplifier,
c). on clive les premiers et deuxième ARN à amplifier, hybridé respectivement sur le premier et le deuxième ADNc, par une enzyme ayant une activité ribonucléase H, pour obtenir des premiers fragments d'ARN à amplifier, hybridés sur le premier ADNc, et des deuxième fragments d'ARN à amplifier, hybridés sur le deuxième ADNc
d). on élonge les extrémités desdits premiers et deuxièmes fragments d'ARN à amplifier par une enzyme de transcritpase inverse et de déplacement de brin, pour obtenir des hybrides ARN-ADN / ADN
e). on obtient des transcrits desdits premiers et deuxième ARN à amplifier, à partir des hybrides ARN-ADN / ADN formés lors de l'étape d) par une enzyme ayant une activité ARN polymerase.

6. Méthode pour générer des transcrits selon la revendication 5 comprenant en outre l'étape suivante :
f. on utilise une première sonde d'hybridation pour détecter les transcrits du premier ARN à amplfier et une deuxième sonde d'hybridation pour détecter les transcrits du deuxième ARN à amplfier

7. Méthode pour générer des transcrits selon l'une quelconque des revendications 5 ou 6 selon laquelle on effectue les étapes a) à e) pendant un temps suffisant pour obtenir un nombre suffisant de transcrits.

8. Méthode pour générer des transcrits selon l'une quelconque des revendications 5 à 7 dans laquelle l'enzyme ayant une activité ARN polymérase est une ARN polymerase de bacteriophage, préférentiellement l'ARN polymérase du bactériophage T7.

## Claims

1. A method for generating transcripts from:
• at least one RNA sequence to be amplified comprising a "primer" region and a region of interest, and
• an amplification primer comprising a promoter region, and a region capable of hybridizing to said "primer" region of the RNA sequence to be amplified,
said method being carried out at constant temperature, and comprising the following steps:
a) said primer is hybridized with the RNA to be amplified,
b) the primer is extended by means of a reverse transcriptase enzymatic activity in order to generate a complementary deoxyribonucleic acid (cDNA) sequence of the RNA to be amplified,
c) the RNA to be amplified, hybridized to said cDNA, is cleaved by means of an enzyme that has a ribonuclease H activity, so as to obtain fragments of RNA to be amplified, hybridized to said cDNA,
d) the ends of said fragments of RNA to be amplified are extended by means of a reverse transcriptase and strand displacement enzyme, so as to obtain RNA-DNA/DNA hybrids,
e) RNA transcripts are obtained from the RNA-DNA/DNA hybrids formed in step d), by means of an enzyme that has an RNA polymerase activity.

2. The method for generating transcripts as claimed in claim 1, also comprising the following step:
f. a hybridization probe specific for said region of interest is used for detecting the RNA transcripts generated.

3. The method for generating transcripts as claimed in either one of claims 1 and 2, according to which steps a) to e) are carried our for a period of time sufficient to obtain a sufficient number of transcripts.

4. The method for generating transcripts as claimed in any one of claims 1 to 3, in which the enzyme that has an RNA polymerase activity is a bacteriophage RNA polymerase, preferably the RNA polymerase of the T7 bacteriophage.

5. A method for generating transcripts from:
• at least a first RNA sequence to be amplified comprising a "primer" region and a region of interest, and a first amplification primer comprising a promoter region and a region capable of hybridizing to said "primer" region of the first RNA sequence to be amplified,
• at least a second RNA sequence to be amplified, different than said first RNA sequence to be amplified, and comprising a "primer" region and a region of interest, and a second amplification primer comprising a promoter region and a region capable of hybridizing to said "primer" region of the second RNA sequence to be amplified,
said method being carried out at constant temperature, and comprising the following steps:
a). said first and second primers are hybridized with said first and second RNAs to be amplified,
b). said first and second primers are extended by means of a reverse transcriptase enzymatic activity in order to generate a first complementary deoxyribonucleic acid (cDNA) sequence of the first RNA to be amplified and a second complementary deoxyribonucleic acid (cDNA) sequence of the second RNA to be amplified,
c). the first and second RNAs to be amplified, hybridized respectively to the first and second cDNAs, are cleaved by means of an enzyme that has a ribonuclease H activity, so as to obtain first fragments of RNA to be amplified, hybridized to the first cDNA, and second fragments of RNA to be amplified, hybridized to the second cDNA,
d). the ends of said first and second fragments of RNA to be amplified are extended by means of a reverse transcriptase and strand displacement enzyme, so as to obtain RNA-DNA/DNA hybrids,
e). transcripts of said first and second RNAs to be amplified are obtained from the RNA-DNA/DNA hybrids formed in step d), by means of an enzyme that has an RNA polymerase activity.

6. The method for generating transcripts as claimed in claim 5, also comprising the following step:
f. a first hybridization probe is used for detecting the transcripts of the first RNA to be amplified and a second hybridization probe is used for detecting the transcripts of the second RNA to be amplified.

7. The method for generating transcripts as claimed in either one of claims 5 and 6, according to which steps a) to e) are carried out for a period of time sufficient to obtain a sufficient number of transcripts.

8. The method for generating transcripts as claimed in any one of claims 5 to 7, in which the enzyme that has an RNA polymerase activity is a bacteriophage RNA polymerase, preferably the RNA polymerase of the T7 bacteriophage.

## Patentansprüche

1. Verfahren zur Herstellung von Transkripten ausgehend von
• mindestens einer zu amplifizierenden RNA-Sequenz, die eine "Primer"-Region und eine interessierende Region umfasst, und
• einem Amplifikationsprimer, der eine Promotorregion und eine Region, die mit der "Primer"-Region der zu amplifizierenden RNA-Sequenz hybridisieren kann, umfasst,
wobei das Verfahren bei konstanter Temperatur durchgeführt wird und man bei dem Verfahren:
a) den Primer mit der zu amplifizierenden RNA hybridisiert,
b) den Primer mit einer reversen Transkriptase-Enzymaktivität verlängert, um eine komplementäre Desoxyribonukleinsäure- (cDNA-) Sequenz der zu amplifizierenden RNA herzustellen,
c) die zu amplifizierende RNA, die an die cDNA hybridisiert ist, durch ein Enzym mit einer Ribonuklease-H-Aktivität spaltet, um zu amplifizierende RNA-Fragmente zu erhalten, die an die cDNA hybridisiert sind,
d) die Enden dieser zu amplifizierenden RNA-Fragmente durch ein reverse-Transkriptase- und Strand-Displacement- Enzym verlängert, um RNA-DNA/DNA-Hybride zu erhalten,
e) die RNA-Transkripte aus den während Schritt d) gebildeten RNA-DNA/DNA-Hybriden durch ein Enzym mit RNA-Polymerase-Aktivität erhält.

2. Verfahren zur Herstellung von Transkripten nach Anspruch 1, bei dem man ferner:
f) eine für die interessierende Region spezifische Hybridisierungssonde verwendet, um die hergestellten RNA-Transkripte nachzuweisen.

3. Verfahren zur Herstellung von Transkripten nach einem der Ansprüche 1 oder 2, wobei die Schritte a) bis e) für eine Zeit durchgeführt werden, die ausreicht, dass eine genügende Anzahl an Transkripten erhalten wird.

4. Verfahren zur Herstellung von Transkripten nach einem der Ansprüche 1 bis 3, wobei das Enzym mit RNA-Polymerase-Aktivität eine Bakteriophagen-RNA-Polymerase, vorzugsweise die RNA-Polymerase des Bakteriophagen T7, ist.

5. Verfahren zur Herstellung von Transkripten ausgehend von
• mindestens einer ersten zu amplifizierenden RNA-Sequenz, die eine "Primer"-Region und eine interessierende Region umfasst, und einem ersten Amplifikationsprimer, der eine Promotorregion und eine Region, die an die "Primer"-Region der ersten zu amplifizierenden RNA-Sequenz hybridisieren kann, umfasst,
• mindestens einer zweiten zu amplifizierenden RNA-Sequenz, die von der ersten zu amplifizierenden RNA-Sequenz verschieden ist und eine "Primer"-Region und eine interessierende Region umfasst, und einem zweiten Amplifikationsprimer, der eine Promotorregion und eine Region, die an die "Primer"-Region der zweiten zu amplifizierenden RNA-Sequenz hybridisieren kann, umfasst,
wobei das Verfahren bei konstanter Temperatur durchgeführt wird und man bei dem Verfahren:
a) die ersten und zweiten Primer mit der ersten und der zweiten zu amplifizierenden RNA hybridisiert,
b) die ersten und zweiten Primer mit einer reversen Transkriptase-Enzymaktivität verlängert, um eine erste komplementäre Desoxyribonukleinsäure- (cDNA-) Sequenz der ersten zu amplifizierenden RNA und eine zweite komplementäre Desoxyribonukleinsäure- (cDNA-) Sequenz der zweiten zu amplifizierenden RNA herzustellen,
c) die erste und die zweite zu amplifizierende RNA, die jeweils an die erste bzw. die zweite cDNA hybridisiert sind, durch ein Enzym mit einer Ribonuklease-H-Aktivität spaltet, um erste zu amplifizierende RNA-Fragmente, die an die erste cDNA hybridisiert sind, und zweite zu amplifizierende RNA-Fragmente, die an die zweite cDNA hybridisiert sind, zu erhalten,
d) die Enden dieser ersten und zweiten zu amplifizierenden RNA-Fragmente durch ein reverse-Transkriptase- und Strand-Displacement- Enzym verlängert, um RNA-DNA/DNA-Hybride zu erhalten,
e) die Transkripte der ersten und zweiten zu amplifizierenden RNA aus den während Schritt d) gebildeten RNA-DNA/DNA-Hybriden durch ein Enzym mit RNA-Polymerase-Aktivität erhält.

6. Verfahren zur Herstellung von Transkripten nach Anspruch 5, bei dem man ferner:
f) eine erste Hybridisierungssonde zum Nachweisen der Transkripte der ersten zu amplifizierenden RNA und eine zweite Hybridisierungssonde zum Nachweisen der Transkripte der zweiten zu amplifizierenden RNA verwendet.

7. Verfahren zur Herstellung von Transkripten nach einem der Ansprüche 5 oder 6, wobei man die Schritte a) bis e) für eine Zeit durchführt, die ausreicht, dass eine genügende Anzahl an Transkripten erhalten wird.

8. Verfahren zur Herstellung von Transkripten nach einem der Ansprüche 5 bis 7, wobei das Enzym mit RNA-Polymerase-Aktivität eine Bakteriophagen-RNA-Polymerase, vorzugsweise die RNA-Polymerase des Bakteriophagen T7, ist.
